(19) **Europäisches Patentamt**

**European Patent Office**

**Office européen des brevets**

(11) **EP 0 695 324 B2**

(12) **NEUE EUROPÄISCHE PATENTSCHRIFT**

(45) Veröffentlichungstag und Bekanntmachung des Hinweises auf die Entscheidung über den Einspruch:
**16.11.2005 Patentblatt 2005/46**

(51) Int Cl.⁷: **C08L 1/02**, D01F 2/00, C08J 3/09

(45) Hinweis auf die Patenterteilung:
**29.10.1997 Patentblatt 1997/44**

(86) Internationale Anmeldenummer:
**PCT/AT1995/000021**

(21) Anmeldenummer: **95906834.7**

(87) Internationale Veröffentlichungsnummer:
**WO 1995/023827 (08.09.1995 Gazette 1995/38)**

(22) Anmeldetag: **01.02.1995**

(54) **VERFAHREN ZUR HERSTELLUNG CELLULOSISCHER FORMKÖRPER**

PROCESS FOR PRODUCING CELLULOSE SHAPED BODIES

PROCEDE DE PRODUCTION DE CORPS MOULES EN CELLULOSE

(84) Benannte Vertragsstaaten:
**AT BE CH DE DK ES FR GB GR IE IT LI LU MC NL PT SE**
Benannte Erstreckungsstaaten:
**LT SI**

(30) Priorität: **01.03.1994 AT 43094**

(43) Veröffentlichungstag der Anmeldung:
**07.02.1996 Patentblatt 1996/06**

(73) Patentinhaber: **LENZING AKTIENGESELLSCHAFT**
**4860 Lenzing (AT)**

(72) Erfinder:
• **KALT, Wolfram**
**A-4860 Lenzing (AT)**
• **FIRGO, Heinrich**
**A-4840 Vöcklabruck (AT)**
• **MÄNNER, Johann**
**A-4852 Weyregg (AT)**
• **MÜLLEDER, Eduard**
**A-4030 Linz (AT)**
• **MANGENG, Bruno**
**A-4020 Linz (AT)**
• **NIGSCH, Arnold**
**A-4840 Vöcklabruck (AT)**
• **SCHWENNINGER, Franz**
**A-4860 Lenzing (AT)**
• **SCHREMPF, Christoph**
**A-4701 Bad Schallerbach (AT)**

(74) Vertreter: **Schwarz, Albin**
**Kopecky & Schwarz**
**Patentanwälte**
**Wipplingerstrasse 32/22**
**1010 Wien (AT)**

(56) Entgegenhaltungen:
**EP-A- 0 427 701**      **EP-A- 0 488 988**
**WO-A-93/11287**      **WO-A-95/08010**
**AT-A- 185 793**      **DD-A- 218 104**
**DD-A- 229 708**      **DD-A- 254 199**
**US-A- 4 324 593**

EP 0 695 324 B2

**Beschreibung**

Die Erfindung betrifft ein Verfahren zur Herstellung cellulosischer Formkörper.

Seit einigen Jahrzehnten wird nach Verfahren zur Herstellung cellulosischer Formkörper gesucht, welche das heute in großem Maßstab angewendete Viskoseverfahren ersetzen sollen. Als eine nicht zuletzt wegen einer besseren Umweltverträglichkeit interessante Alternative hat sich dabei herauskristallisiert, Cellulose ohne Derivatisierung in einem organischen Lösungsmittel aufzulösen und aus dieser Lösung Formkörper, z.B. Fasern und Folien, zu extrudieren. Solcherart extrudierte Fasern erhielten von der BISFA (The International Bureau for the Standardization of man made fibers) den Gattungsnamen Lyocell. Unter einem organischen Lösungsmittel wird von der BISFA ein Gemisch aus einer organischen Chemikalie und Wasser verstanden.

Es hat sich herausgestellt, daß sich als organisches Lösungsmittel insbesondere ein Gemisch aus einem tertiären Aminoxid und Wasser sehr gut zur Herstellung von cellulosischen Formkörpern eignet. Als Aminoxid wird dabei in erster Linie N-Methylmorpholin-N-oxid (NMMO) verwendet. Andere Aminoxide sind z.B. in der EP-A - 0 533 070 beschrieben. Ein Verfahren zur Herstellung formbarer Celluloselösungen ist z.B. aus der EP-A - 0 365 419 bekannt.

Die Cellulose wird aus der Celluloselösung in einem wäßrigen Fällbad gefällt. Dabei reichert sich das Fällbad mit Aminoxid an. Für die Wirtschaftlichkeit des Verfahrens ist es von entscheidender Bedeutung, daß das Aminoxid nahezu vollständig zurückgewonnen und wiederverwendet wird. Das Aminoxidverfahren weist somit die folgenden 3 Hauptschritte auf:

(A) Auflösen von Cellulose in einer wäßrigen Lösung eines tertiären Aminoxids, insbesondere N-Methylmorpholin-N-oxid (NMMO) um eine formbare Celluloselösung zu bilden,

(B) Formen der Celluloselösung und Führen der geformten Celluloselösung in ein wäßriges Fällbad, in welchem die Cellulose gefällt wird, wodurch ein Formkörper und ein gebrauchtes Fällbad gebildet werden,

(C) Regenerieren, d.h. Reinigen und Konzentrieren, des gebrauchten Fällbades, wobei eine regenerierte, wäßrige Aminoxidlösung gebildet wird, die im Schritt (A) erneut zur Auflösung von Cellulose eingesetzt wird.

Unter dem Begriff "Regenerieren" werden jegliche Maßnahmen verstanden, die dazu dienen, das Fällbad zu einer wäßrigen Aminoxidlösung aufzubereiten, die wieder im Schritt (A) eingesetzt werden kann. Solche Maßnahmen sind z.B. Reinigen, Behandeln mit Ionenaustauschern, Konzentrieren etc.

Im Fällbad reichert sich nicht nur Aminoxid sondern auch Abbauprodukte der Cellulose und des Aminoxids an. Diese können stark gefärbt sein und die Qualität der hergestellten Formkörper beeinträchtigen, wenn sie nicht aus dem Fällbad entfernt werden. Zusätzlich können sich im Fällbad auch Metallspuren anreichern, die zu einer Verminderung der Prozeßsicherheit führen.

Für das Entfernen dieser Abbauprodukte sind aus der Literatur einige Vorschläge bekannt:

Die DD-A 254 199 beschreibt ein Verfahren zur Reinigung wäßriger Lösungen von NMMO, gemäß welchem die Lösung Anionenaustauscher passiert, wobei in einer ersten Stufe der Anionenaustauscher ein mit tertiären Aminogruppen vom Typ -$CH_2N(CH_3)_2$ besetztes Austauscherharz eines Styrol/Divinylbenzol-Copolymorisates enthält und in einer zweiten Stufe als funktionelle Gruppen quaternäre Ammoniumgruppen vom Typ -$CH_2N(CH_3)_3OH$ enthält. Es wird beschrieben, daß die zu reinigende NMMO-Lösung zu Beginn der Reinigung dunkel, nach der ersten Stufe braun bis gelb und nach der zweiten Stufe hellgelb bis wasserklar ist.

Ein Nachteil dieses Verfahrens besteht darin, daß die solcherart behandelten Lösungen einen hohen pH-Wert aufweisen, der in weiterer Folge zu einem erhöhten Reinigungsaufwand führt. Dazu kommt noch, daß bei diesem vorbekannten Verfahren Alkali- und Erdalkalikationen, sowie teilweise basische Abbauprodukte (Morpholin, N-Methylmorpholin und andere Verbindungen) nicht aus der Lösung entfernt werden. Die Metallionen bzw. Alkali- und Erdalkalimetallionen führen zu unerwünschten Ablagerungen und Verkrustungen, zu ungelösten störenden Stoffen in der Lösung sowie zu einer Verringerung der Prozeßsicherheit. Es ist zwar möglich, diese Stoffe durch Zugabe eines Fällungsmittels mit anschließender Filtration oder anderen Trennmitteln zu entfernen, diese Arbeitsgänge tragen jedoch wiederum zusätzliche Chemikalien ein bzw. bedeuten einen zusätzlichen technischen Aufwand.

In der EP-A - 0 427 701 ist ein Verfahren zur Reinigung von wäßrigen Aminoxidlösungen beschrieben, gemäß welchem die Reinigung in einem einstufigen Verfahren mit einem Anionenaustauscher durchgeführt wird, der als funktionelle Gruppen ausschließlich quaternäre Tetraalkylammoniumgruppen der Formeln -$CH_2N^+(CH_3)_3X^-$ oder -$CH_2N^+(CH_3)_2(CH_2OH)]X^-$ aufweist, wobei $X^-$ das Anion einer anorganischen oder organischen Säure darstellt, worauf der Anionenaustauscher mit einer wäßrigen sauren Lösung regeneriert wird. Das Anion $X^-$ stammt vorzugsweise von einer flüchtigen Säure, insbesondere Kohlensäure, Ameisensäure oder Essigsäure. Diese Säuren werden auch zur Regenerierung des Anionenaustauschers vorgeschlagen.

In der Internationalen Patentanmeldung WO93/11287 wird vorgeschlagen, die Regenerierung des Anionenaustauschers mit einer wäßrigen Lösung einer starken anorganischen Säure und anschließend mit Natronlauge durchzuführen. Es wird ferner vorgeschlagen, die Lösung vor, oder bevorzugt nach, dem Passieren des Anionenaustauschers über einen Kationenaustauscher zu führen. Es wird beschrieben, daß bei Verwendung eines stark basischen Anionenaustauschers die durch das Überleiten der zu reinigenden Lösung entstehende Färbung des Austauscherharzes so stark sei, daß eine bloße Regenerierung mit Natronlauge nicht ausreichend ist, um das Harz wieder zu entfärben. Um die Kapazität des Harzes aufrechtzuerhalten, muß es daher zusätzlich mit einer starken anorganischen Säure behandelt werden.

Die in der WO93/11287 beschriebene Verfahrensweise führt zu einem gesteigerten Chemikalieneinsatz und zwingt zum Verwenden stark reizender Substanzen, wie z.B. Salzsäure. Zusätzlich ist Beispiel 5 der WO93/11287 zu entnehmen, daß selbst bei Anwendung dieses Verfahrens die Entfärbungskapazität des Anionenaustauschers nach 10 Durchläufen auf beinahe die Hälfte des ursprünglichen Wertes gesunken ist.

Ein Nachteil des Aminoxidverfahrens gegenüber dem Viskoseverfahren ist die geringe thermische Stabilität der Aminoxide und insbesondere der Celluloselösungen. Darunter ist zu verstehen, daß in den Celluloselösungen bei den erhöhten Temperatur der Verarbeitung (etwa 110-120°C) unkontrollierbare, stark exotherme Zersetzungsprozesse ausgelöst werden können, die unter Entwicklung von Gasen zu heftigen Verpuffungen oder Explosionen und in weiterer Folge auch zu Bränden führen können.

Über die geringe thermische Stabilität der Celluloselösung ist in der Literatur nur wenig bekannt. Auf das Phänomen ist von Buijtenhuijs et al. 1986 erstmals deutlich hingewiesen worden. Insbesondere bei Anwesenheit von Metallionen können in bestimmten Fällen die Zersetzungsreaktionen in der Spinnmasse durchgehen. Metallionen sind aber in der Lösung aufgrund des metallischen Aufbaues der Anlagenteile nie auszuschließen.

Das Durchgehen kann selbst durch Zugabe des heute vielfach verwendeten Stabilisators Gallussäurepropylester (GPE) nicht verhindert werden (Buijtenhuijs et al., 1986). Im Gegenteil: wie Untersuchungen ergeben haben, erhöhen GPE und auch andere aromatische Hydroxyverbindungen mit guten Komplexierungseigenschaften unter besonderen Voraussetzungen die thermische Instabilität der NMMO-Celluloselösung bei Anwesenheit von Metallen sogar noch; d.h., daß GPE das gefährliche Durchgehen bzw. die Explosionen (mit)initiieren kann. Dies ist in der österreichischen Patentanmeldung A 1857/93, die am 15. Oktober 1994 veröffentlicht wurde, beschrieben.

Aus der US-A - 4,324,593 ist ein Verfahren zur Herstellung einer formbaren Lösung bekannt, welche Cellulose enthält, die in einem Lösungsmittel gelöst ist. Das Lösungsmittel enthält ein tertiäres Aminoxid und eine Verbindung, welche die Lösungsgeschwindigkeit der Cellulose erhöht. Als derartige Verbindungen werden insbesondere primäre, sekundäre, tertiäre Amine, wäßriger Ammoniak und Alkalihydroxide genannt, von denen tertiäre Amine bevorzugt sind. Die Autoren des Patentes vermuten, daß die beschleunigende Wirkung dieser Verbindungen primär darauf zurückzuführen sind, daß sie den pH der Lösung erhöhen. Ein Nachweis für die Richtigkeit dieser Vermutung wird jedoch nicht erbracht, und es wird auch nicht angegeben, welchen pH die Lösung aufweisen sollte. Lediglich im Anspruch 27 der US-A - 4,324,593 ist ganz allgemein erwähnt, daß die beschleunigende Verbindung einen pH von größer als 7 haben sollte, und in den Beispielen XIV und XV wird der pH einer Mischung bestehend aus festem tertiären Aminoxid, Cellulose und Wasser mit Natriumhydroxid bzw. wäßrigem Ammoniak auf einen pH von 14 bzw. 12,3 eingestellt.

In der US-A - 4,324,593 wird vorgeschlagen, die beschleunigende Verbindung dem Lösungsmittel in einer solchen Menge zuzugeben, daß sie bis zu 20% der Masse der fertigen Lösung ausmacht, wobei die Menge im Einzelfall vom verwendeten Aminoxid abhängen soll.

Zur Verhinderung des Abbaues von NMMO und Cellulose ist es aus der DD-A - 0 218 104 bekannt, dem Aminoxid eine oder mehrere basische Substanzen in Mengen zwischen 0,1 und 10 Mol%, bezogen auf die Celluloselösung, zuzusetzen. Als basische Substanz werden Alkalihydroxide, z.B. NaOH, basisch reagierende Salze, z.B. $Na_2CO_3$, sowie organische Stickstoffbasen empfohlen.

Die vorliegende Erfindung setzt sich zum Ziel, das Aminoxidverfahren derart weiterzuentwickeln, daß es auf einfach Weise möglich ist, sowohl die thermische Stabilität der Celluloselösung zu erhöhen als auch den Abbau an Cellulose möglichst gering zu halten. Es ist insbesondere ein Ziel der Erfindung, die oben genannten Abbauprodukte und Verunreinigungen, die sich im Fällbad anreichern, aus dem Verfahren zu entfernen.

Das erfindungsgemäße Verfahren zur Herstellung cellulosischer Formkörper weist die folgenden Schritte auf:

(A) Auflösen von Cellulose in einer wäßrigen Lösung eines tertiären Aminoxids, insbesondere N-Methylmorpholin-N-oxid (NMMO), um eine formbare Celluloselösung zu bilden,

(B) Formen der Celluloselösung und Führen dergeformten Celluloselösung in ein wäßriges Fällbad, in welchem die Cellulose gefällt wird, wodurch ein Formköper und ein gebrauchtes Fällbad gebildet werden,

(C) Regenerieren des gebrauchten Fällbades, wobei eine regenierte, wäßrige Aminoxidlösung gebildet wird, die im Schritt (A) erneut zur Auflösung von Cellulose eingesetzt wird,

und ist dadurch gekennzeichnet,
daß eine regenerierte, wäßrige Aminoxidlösung im Schritt (A) eingesetzt wird, die einen pH-Wert in einem Bereich aufweist, dessen obere und dessen untere Grenze, in Abhängigkeit von der Konzentration an tertiärem Aminoxid, durch die Gleichung

$$pH = -0.0015 \times A^2 + 0{,}2816 \times A + 1$$

definiert werden, wobei A die Konzentration an tertiärem Aminoxid in der wäßrigen Lösung, ausgedrückt in % Masse der wäßrigen Lösung, ist und die Bedingung

$$40\% \leq A \leq 86\%,$$

vorzugsweise

$$70\% \leq A \leq 80\%,$$

erfullt, und die obere Grenze den Wert 1,00 und für die untere Grenze den Wert -1.80, vorzugsweise .1.00. besitzt.

Ein weiterer Aspekt der vorliegenden Erfindung ist ein Verfahren zur Herstellung cellulosischer Formkörper, welches die obigen Schritte (A) bis (C) aufweist und dadurch gekennzeichnet ist, daß eine regenerierte, wäßrige Aminoxidlösung im Schritt (A) eingesetzt wird, die einen pH-Wert in einem Bereich zwischen 11,5 und 13,5 aufweist.

Die Erfindung beruht auf der Erkenntnis, daß die Stabilität der Callutoselösung in hohem Ausmaß davon abhängt, welchen pH-Wert die zur Suspensionsbereitung verwendete regenerierte, wäßrige Aminoxidlösung aufweist. Im Sinno der vorliegenden Patentanmeldung wird eine Celluloselösung als umso stabiler angesehen, wenn ihre mittels DSC (Differential Scanning Calorimetry) bestimmte thermische Stabilität möglichst hoch ist und gleichzeitig möglichst wenig Cellulose abgebaut wird, was sich in einem hohen Pelymerisationsgradder Cellulose und in einer hohen Viskosität der Celluloselösung äußert

Es hat sich gezeigt, daß sich die thermische Stabilität von Celluloselösungen sprunghalt vorbossert, wenn zu ihrer Horsteilung eine wäßrige Aminoxidlösung eingesetzt wird, deren pH 10,5 oder großer ist. Am thermodynamisch stabilsten erweisen sich Celluloselösungen, die aus einer wäßrigen Amin. oxidlösung hergestellt werden, deren pH im Bereich von 11,5 bis 12,5 hegl, wobei bei pH 12,0 die maximale thermische Stabilität gegeben ist.

Dia Erfindung beruht ferner auf der Erkenntnis, daß ab einem pH von mindestens 10.5 der Polymerisationsgrad der Cellulose deutlich weniger abnimmt, d h. deutlich weniger Cellulose abgebaut wird. Das Maximum dieser günstigen Wirkung liegt ebenfalls im pH-Bereich von 11,5 bis 12,5, vras bedeutet, daß die höchste thermische Stabilität der Celluloselösung und die höchste Stabilität der Cellulose gegen Abbau im gleichen pH-Bereich gegeben sind und somit zusammenfallen.

Auch die Viskositätsmessungen, die an Celluloselösungen durchgeführt wurden, zeigen, daß die Cellulose offenbar dann am wenigsten abgebaut wird wenn die eingeseizte Aminoxidlösung einen pH von mindestens 10,5 aufweist. Unterhalb von 10,5 fällt die Viskosital stark ab.

Das erfindungsgemäße Verfahren wird demgemäß bevorzugt so ausgeführt, daß die regenenerte, wäßrige Aminoxidlösung, die im Schnit (A) eingesetzt wird, einen pH-Wert im Bereich von 10,5 und 13,5, mehr bevorzugt im Bereich von 11,5 und 13,5 und am meisten bevorzugt im Bereich von 11,5 und 12,5, aufweist.

Der pH-Wert der regonenerten wäßrigen Aminoxidlosung kann auf einfache Weise eingestellt werden, indem das gebrauchte Fällbad mit einem alkalischen Anionenaustauscher und gegebenenfalls anschließend mit einem sauren Kauoncnaustauschor in Kontakt gebracht wird.

Es hat sich ferner als vorteihall erwiesen, den pH-Wert der regenenerten, wäßrigen Aminoxidlösung einzustellen indern das gebrauchte Fällbad mit einem mit alkalischen Gruppen modifizierten Adsorberharz, anschließend mit einem alkalischen Anionenaustauscher und zumindest teilweise mit einem sauren Kationenaustauscher in Kontakt gebracht wird.

Es kann das gesamte Fällbad oder nur ein Teilstrom mit dem modifizierten Adsorberharz bzw. den Ionenauslauschem in Kontakt gebracht werden, was naturgemäß vom Ausmaß der Verlärbung, dem Gehalt an Kationen und Anionen und dem gewünschlen End-pH der regenerierten Lösung abhängt.

Es hat soch gezeigt, daß durch die Verwendung von Adsorberharzen und lonenaustauschern nicht nur der pH-Wert der NMMO-Lösung auf einfache Weise eingestellt werden kann, sondern daß auch die im Fällbad angereicherten Abbauprodukte, die zu Verfärbungen führen besonders wirkungsvoll entfernt werden können. Zusätzlich ist durch die Ausführungsform mit dem Adsorberharz gewährleistet, daß die Regenerierung des nachfolgenden Anionenaustauschers im wesentlichen ohne zusätzliche Chemikalien, wie z.B. stark reizende Säuren, möglich ist. Das erfindungsgemäß verwendete Adsorberharz unterscheidet sich von dem in der DD-A 254 899 in der ersten Stute eingesetzten schwach basischen Anionenaustauscher dadurch, daß es such nicht um einen schwach basischen Anionenaustauscher um eigentlichen Sinn handelt, sondern um ein Harz, dessen Aulgabe nicht der Austausch, sondern die Adsorption von Substanzen ist. Ein Adsorberharz weist aus diesem Grund auch eine besondere makroporöse Porenstrukfur auf. Zusätzlich ist das Harz in geringerem Ausmaß, als dies bei einem Anionenaustauscher üblich ist, mit schwach basischen Gruppen modifiziert.

Es hat sich nun gezeigt, daß der Einsatz eines solchorart medilizierten Adsorborharzes zur Regeneriorung des Fallbades nicht nur zur elfizienleren Enlternung von gefärbten Stoffen aus der Lösung führt, sondom auch die Regenerierbarkeit des Harzes deutlich besser ist, als jene der in der Literatur beschriebenen Aniononaustauschor

Es hat sich ferner herausgestellt, daß durch den Einsatz eines Adsorberharzes irreversible Verlarbungen des nachgeschatleten Anionenaustauschers vermieden werden und dadurch kein über die Angaben des Herstellers hinausgehender wesentlicher Kapazitätsverlust im Anionenaustauscher auftritt. Dies ermöglicht eine ausreichende Regenerierung das Anionenaustauschers mit Alkalilauge, z.B. mit Natronlauge. Der zusätzliche Einsatz von starken Säuren kann demgemäß vermieden werden. Eine weitere bevorzugte Ausführungsform des erfindungsgemäßen Verfahrens verwendet daher einen Anionenaustauscher, der ausschließlich mit Alkalilauge und/oder flüchtigen organischen Säuren regeneriert wurde.

Durch das Nachschalten des Kationenaustauschers nach dem Anionenaustauscher kann auf einfache Weise und ohne zusätzliche chemische oder mechanische Hilfsmittel erreicht werden, daß der aufgrund des Kontaktes mit dem Anionenaustauscher entstandene und den pH-Wert stark erhöhende Hydroxidüberschuß kompensiert wird. Zusätzlich werden Kationen aus der Lösung entfernt.

Vorteilhafterweise weist das mit basischen Gruppen modifizierte Adsorberharz tertiäre Aminogruppen als funktionelle Gruppen auf. Tertiäre Aminogruppen, wie z.B. Gruppen des Typs -$CH_2N(R)_2$, wobei R Alkyl, Hydroxyalkyl etc. ist, sind als schwach alkalische Gruppen mit ionenaustauschender Wirkung bekannt und verstärken in Kombination mit der adsorptiven Wirkung des Adsorberharzes die entfärbende Wirkung.

Weiters weist der Anionenaustauscher vorteilhafterweise als funktionelle Gruppen quarternäre Ammoniumgruppen auf. Diese Gruppen, z.B. vom Typus -$CH_2N^+(CH_3)_3$ oder -$CH_2N^+[(CH_3)_2(CH_2OH)]$, sind als stark basische funktionelle Gruppen mit ionenaustauschender Wirkung bekannt und erfüllen im erfindungsgemäßen Verfahren in besonders effizienter Weise die Aufgabe, störende Anionen aus der Lösung zu entfernen.

Der Kationenaustauscher weist vorteilhaft Sulfonsäuregruppen als funktionelle Gruppen auf. Sulfonsäuregruppen sind in Kationenaustauschern bekannt.

Die Stabilität der Celluloselösung kann ferner zusätzlich erhöht werden, wenn vor, während oder nach dem Regenerieren des Fällbades neben der alkalischen Substanz auch eine antioxidativ wirkende Substanz, also ein Antioxidans, eingebracht wird.

Der Begriff "Antioxidans" wird so verstanden, daß damit alle Stoffe und Stoffgemische umfaßt werden, die einem oxidativen und radikalischen Abbau der Cellulose entgegenwirken. Auch Radikalfänger und Reduktionsmittel fallen naturgemäß unter diesen Begriff. Derartige Stoffe sind z.B. die aus der DE-A-2 000 082 bekannten mehrwertigen Phenole, mehrwertigen Oxycarbonsäuren, Trioxybenzole etc. Bevorzugte Antioxidantien sind Tannine und jene Stoffe, die in der EP-B - 0 047 929 genannt sind, d.h. Glycerinaldehyd und/oder ein oder mehrere organische Verbindungen, die wenigstens vier Kohlenstoffatome und wenigstens zwei konjugierte Doppelbindungen und wenigstens zwei Hydroxyl- und/oder Aminogruppen mit wenigstens einem Wasserstoffatom besitzen. Insbesondere bevorzugt sind Brenzkatechin, Pyrogallol, Gallussäure, Gallussäuremethylester, -ethylester, -propylester und -isopropylester. Auch Hydrochinon und Anthrachinon bzw. strukturanaloge Verbindungen sowie Derivate können als Antioxidans eingesetzt werden.

Die Erfindung wird mit den folgenden Beispielen noch näher erläutert, wobei als Celluloselösungen jeweils Kneterspinnmassen dienten, zu deren Herstellung wäßrige NMMO-Lösungen mit pH-Werten im Bereich von 9,5 bis 13,5 verwendet wurden. Sämtliche Prozentangaben sind auf die Masse bezogen.

Die Messungen des pH-Wertes erfolgten jeweils mit einer pH-Elektrode in Form einer Einstabmeßkette (Metrohm 6.0210.100) bei 50°C mit einer Einstellzeit von 90 Sekunden.

(1) Herstellung der Kneterspinnmassen

Die Kneterspinnmassen wurden nach dem folgenden allgemeinen Verfahren hergestellt:

In ein 250ml Becherglas wurden Gallussäurepropylester und Hydroxylamin als Stabilisatoren in Mengen eingewogen, die 0,03% bzw. 0,05% des eingesetzten Zellstoffs entsprach. Danach wurden 221 g einer wäßrigen 72,46%igen NMMO-Lösung mit einem pH im Bereich von 9,5 und 13,5 (der pH wurde mit NaOH und/oder $H_2SO_4$ eingestellt) zugegeben, 5 Minuten lang bei Raumtemperatur gerührt, und anschließend wurde die erhaltene Lösung in einen Laborkneter gegeben.

Das Becherglas wurde mit 25,5 g faserig gemahlenem, luftgetrockneten Zellstoff (ca. 94%) ausgetrocknet, und danach wurde der Zellstoff ebenfalls in den Kneter gegeben.

Das Gemenge wurde 15 Minuten bei Raumtemperatur und 250 mbar suspendiert und anschließend aufgeheizt (Thermostateinstellung: 130°C). Bei ca. 90°C destillierte der erste Tropfen Wasser ab, was den eigentlichen Lösungsbeginn anzeigt. 5 Minuten später wurde der Unterdruck in entsprechenden Zeitintervallen um jeweils um 25 mbar bis auf 50 mbar vergrößert. Das Ende des Lösungsvorganges war nach ca. 1 Stunde erreicht.

Nach diesem allgemeinen Verfahren wurden 7 Spinnmassen aus 7 wäßrigen NMMO-Lösungen hergestellt, wobei die einzelnen NMMO-Lösungen folgende pH-Werte aufwiesen: 9,5, 10,5, 11,0, 11,5, 12,0, 12,5, und 13,5.

(2) Thermische Stabilität der Spinnmassen

Die thermische Stabilität der Spinnmassen wurde sowohl für frisch hergestellte Spinnmassen bestimmt, als auch für Spinnmassen, welche vorher 20 Stunden auf 110°C erhitzt worden waren.

Die Untersuchungen zur thermischen Stabilität wurden gemäß Buijtenhuijs et al. (The Degradation and Stabilization of Cellulose Dissolved in N-Methylmorpholin-N-Oxide (NMM), in "Das Papier", 40. Jahrgang, Heft 12, Seiten 615-619, 1986) mittels der DSC-(differential scanning calorimetry)-Technik (Gerät: Mettler Druck DSC Thermosystem 4000) durchgeführt, wobei das in der östereichischen Patentanmeldung A1857/93 beschriebene Verfahren angewendet wurde.

Konfiguration des Druck-DSC: Für Steuerung und Auswertung: TA-Processor TC11; Auswertungssoftware: TA72AT.2; Messung: Druck DDK Meßzelle DSC27HP; eingesetzter Drucker: Epson FX 850.

Versuchsbedingungen:

Die zu untersuchende Spinnmasse (5,8 mg $\pm$ 0,3 mg) wird im festen, ausgekühlten Zustand in einen mehrfach gelochten Alu-Tiegel (offenes System) eingewogen und in der Folge oberflächlich mit einem homogenen Gemisch aus 9 Gewichtsteilen $Fe_2O_3$ (Hersteller Aldrich, Art.Nr. 3924) und 1 Gewichtsteil metallischem Kupfer (Hersteller Merck, Art.Nr. 2715), im Verhältnis 2:1 (2 Teile Spinnmasse : 1 Teil Gemisch) in innigen Kontakt gebracht.

Zur Vornahme der DSC-Messung wurde die Meßkammer nach Einbringen des Alu-Tiegels mit einem Druck von 20 bar Stickstoff beaufschlagt. Danach wurde mit einer Geschwindigkeit von 10°C/min auf eine Temperatur von 112°C (Startpunkt 40°C) aufgeheizt. Im Anschluß daran wurde die Probe über einen Zeitraum von maximal 120 min auf 112°C gehalten und während dieser Zeit die DSC-Kurve aufgenommen. Die beiden Programmteile Aufheizen auf 112°C und Halten bei dieser Temperatur wurden im Prozessor des DSC-Gerätes abgespeichert und von diesem stets unter gleichen Bedingungen verknüpft.

Als Initialisierungspunkt wurde in der DSC-Kurve jener Zeitwert bestimmt, der ein erstes Ansteigen in den exothermen Bereich anzeigt. Als "Onset" wurde jene Zeit festgelegt, bei welcher sich die resultierende Gerade der Extrapolation der Basislinie vor dem Effekt mit der Tangente an die durch den Effekt verursachte Kurve schneidet.

Die nachfolgende Tabelle 1 stellt den pH-Wert der zur Herstellung der Kneterspinnmasse verwendeten NMMO-Lösung den jeweiligen Initialisierungspunkten (IP, in Minuten) und Onset-Punkten (OP, in Minuten) gegenüber, wobei sich IP(th.) und OP(th.) auf die Werte von Spinnmassen beziehen, die vor der Untersuchung der oben erwähnten thermischen Behandlung unterworfen worden waren.

Tabelle 1

| pH-Wert | IP | OP | IP(th.) | OP(th.) |
|---|---|---|---|---|
| 9,5 | 7 | 11 | 0 | 0 |
| 10,5 | 16 | 19 | 0 | 0 |
| 11,0 | 41 | 61 | 20 | 31 |
| 11,5 | 56 | 72 | 29 | 38 |
| 12,0 | 57 | 77 | 38 | 50 |
| 12,5 | 60 | 77 | 30 | 41 |
| 13,5 | 60 | 80 | 26 | 36 |

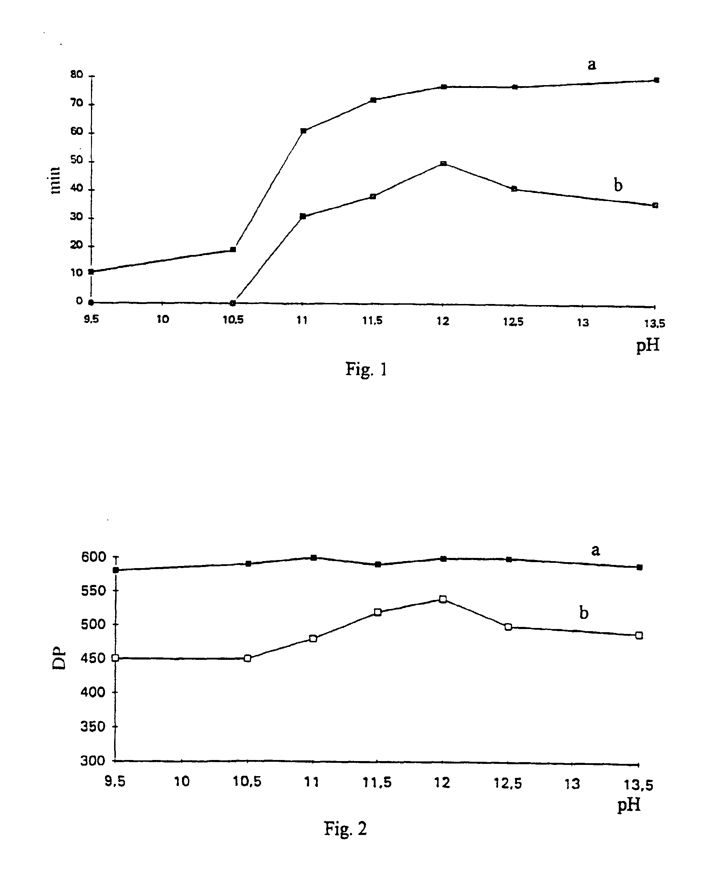

Figur 1 zeigt den gefundenen Zusammenhang graphisch, wobei als Abszisse der pH-Wert der eingesetzten NMMO-Lösung und als Ordinate der Onsetpunkt (in Minuten) aufgetragen sind. Die Kurve "a" zeigt das DSC-Verhalten von Spinnmassen, die keiner thermischen Vorbehandlung unterzogen wurden, und die Kurve "b" zeigt das DSC-Verhalten von thermisch vorbehandelten Spinnmassen.

Den in der Tabelle 1 aufgelisteten Daten bzw. der Figur 1 ist zu entnehmen, daß ab dem pH-Wert von 10,5 der verwendeten NMMO-Lösung die thermische Stabilität der erhaltenen Spinnmasse sprunghaft ansteigt, und daß bei pH 11,5, insbesondere bei thermisch vorbehandelten Spinnmassen, die Stabilität neuerlich zunimmt. Die größte Stabilität ist bei einem pH von etwa 12,0 zu erkennen.

(3) Polymerisationsgrad (DP) der Cellulose

Die nachfolgende Tabelle 2 stellt den Polymerisationsgrad der gelösten Cellulose vor und nach einer thermischen Behandlung der Kneterspinnmasse (20 Stunden bei 110 °C) dem pH-Wert der zur Herstellung der Kneterspinnmasse verwendeten NMMO-Lösung gegenüber.

Tabelle 2

| pH-Wert | DP | DP(th.) |
|---|---|---|
| 9,5 | 580 | 450 |
| 10,5 | 590 | 450 |
| 11,0 | 600 | 480 |
| 11,5 | 590 | 520 |
| 12,0 | 600 | 540 |
| 12,5 | 600 | 500 |
| 13,5 | 590 | 490 |

Die in der Tabelle 2 aufgelisteten Ergebnisse sind in der Figur 2 graphisch dargestellt, wobei die Kurve "a" den DP der Cellulose in den frisch hergestellten Spinnmassen zeigt und die Kurve "b" den DP der Cellulose in Spinnmassen, die der thermischen Behandlung unterzogen wurden. An der Kurve "a" ist zu erkennen, daß der Polymerisationsgrad der Cellulose in frischer Spinnmasse vom pH-Wert des verwendeten NM-

MO praktisch nicht abhängt. Nach der thermischen Behandlung (Kurve "b") zeigt sich aber, daß der Polymerisationsgrad dann weniger abnimmt, wenn der pH der verwendeten NMMO-Lösung zwischen 10,5 und 13,5 liegt, wobei die geringste Abnahme wiederum bei pH 12,0 zu beobachten ist.

(4) Anwendung eines Adsorberharzes und von Ionenaustauschern zur Einstellung des pH-Wertes und zur Reinigung

Eine NMMO-hältige, wäßrige Flüssigkeit, die aus einem gebrauchten Fällbad und anderen Prozeßwässern des NMMO-Verfahrens bestand und etwa 15% NMMO enthielt, wurde zunächst über ein Adsorberharz des Typus XUS 40285.00 (DOWEX), welches mit tertiären Aminogruppen als funktionelle Gruppen modifiziert war, geführt. Dieses Adsorberharz wurde zyklenweise mit verdünnter Natronlauge regeniert und mit Wasser neutral gewaschen.

Die durch das Adsorberharz geführte Flüssigkeit wurde anschließend über einen Anionenaustauscher vom Typ LEWATIT MP 500 (BAYER) geführt. Dieser Anionenaustauscher enthält quaternäre Ammoniumgruppen als funktionelle Gruppen. Der Anionenaustauscher wurde mit verdünnter Natronlauge regeneriert und mit Wasser neutral gewaschen. Es zeigte sich, daß auch nach mehreren Zyklen keine über die Angaben des Herstellers hinausgehende Kapazitätsverminderung am Anionenaustauscher auftrat.

Anschließend wurde ein Teil der Lösung über einen Kationenaustauscher des Typus LEWATIT SM (BAYER), welcher Sulfonsäuregruppen als funktionelle Gruppen enthielt, geführt. Nach dieser Behandlung wurde dieser Teil mit dem restlichen Teil der Lösung, der nicht über den Kationenaustauscher geführt worden war, vereinigt. Nach Aufkonzentrierung auf einen NM-MO-Gehalt von 72% wies die regenerierte NM-MO-Lösung einen pH von ca. 12,0 auf. In dieser NM-MO-Lösung konnten störende Substanzen praktisch nicht mehr nachgewiesen werden bzw. waren lediglich in Mengen vorhanden, die nicht störend sind.

## Patentansprüche

1. Verfahren zur Herstellung cellulosischer Formkörper, welches Verfahren folgende Schritte aufweist:

   (A) Auflösen von Cellulose in einer wäßrigen Lösung eines tertiären Aminoxids, insbesondere N-Methylmorpholin-N-oxid (NMMO), um eine formbare Celluloselösung zu bilden,
   (B) Formen der Celluloselösung und Führen der geformten Celluloselösung in ein wäßriges Fällbad, in welchem die Cellulose gefällt wird, wodurch ein Formköper und ein gebrauchtes Fällbad gebildet werden,

   (C) Regenerieren des gebrauchten Fällbades, wobei eine regenierte, wäßrige Aminoxidlösung gebildet wird, die im Schritt (A) erneut zur Auflösung von Cellulose eingesetzt wird,

   **dadurch gekennzeichnet,**
   **daß** eine regenerierte, wäßrige Aminoxidlösung im Schritt (A) eingesetzt wird, die einen pH-Wert in einem Bereich aufweist, dessen obere und dessen untere Grenze, in Abhängigkeit von der Konzentration an tertiärem Aminoxid, durch die Gleichung

   $$pH = - 0,0015 \times A^2 + 0,2816 \times A + f$$

   definiert werden, wobei A die Konzentration an tertiärem Aminoxid in der wäßrigen Lösung, ausgedrückt in % Masse der wäßrigen Lösung, ist und die Bedingung

   $$40\% \leq A \leq 86\%,$$

   vorzugsweise

   $$70\% \leq A \leq 80\%,$$

   erfüllt, und f für die obere Grenze den Wert 1,00 und für die untere Grenze den Wert -1,80, vorzugsweise -1,00, besitzt.

2. Verfahren zur Herstellung cellulosischer Formkörper, welches Verfahren folgende Schritte aufweist:

   (A) Auflösen von Cellulose in einer wäßrigen Lösung eines tertiären Aminoxids, insbesondere N-Methylmorpholin-N-oxid (NMMO), um eine formbare Celluloselösung zu bilden,
   (B) Formen der Celluloselösung und Führen der geformten Celluloselösung in ein wäßriges Fällbad, in welchem die Cellulose gefällt wird, wodurch ein Formköper und ein gebrauchtes Fällbad gebildet werden,
   (C) Regenerieren des gebrauchten Fällbades, wobei eine regenierte, wäßrige Aminoxidlösung gebildet wird, die im Schritt (A) erneut zur Auflösung von Cellulose eingesetzt wird,

   **dadurch gekennzeichnet,**
   **daß** eine regenerierte, wäßrige Aminoxidlösung im Schritt (A) eingesetzt wird, die einen pH-Wert in einem Bereich zwischen 11,5 und 13,5 aufweist.

3. Verfahren nach Anspruch 1, **dadurch gekennzeichnet, daß** die regenerierte, wäßrige Aminoxidlösung, die im Schritt (A) eingesetzt wird, ein pH-Wert im Bereich von 10,5 und 13,5 aufweist.

4. Verfahren nach Anspruch 1, **dadurch gekennzeichnet, daß** daß die regenerierte, wäßrige Aminoxidlösung, die im Schritt (A) eingesetzt wird, ein pH-Wert im Bereich von 11,5 und 13,5 aufweist.

5. Verfahren nach einem der Ansprüche 1 bis 2, **dadurch gekennzeichnet, daß** die regenerierte, wäßrige Aminoxidlösung, die im Schritt (A) eingesetzt wird, ein pH-Wert im Bereich von 11,5 und 12,5 aufweist.

6. Verfahren nach einem der Ansprüche 1 bis 5, **dadurch gekennzeichnet, daß** der pH-Wert der regenerierten, wäßrigen Aminoxidlösung eingestellt wird, indem das gebrauchte Fällbad mit einem alkalischen Anionenaustauscher, welcher eine alkalische Substanz in das Fällbad einbringt, und gegebenenfalls anschließend mit einem sauren Kationenaustauscher in Kontakt gebracht wird.

7. Verfahren nach einem der Ansprüche 1 bis 5, **dadurch gekennzeichnet, daß** der pH-Wert der regenerierten, wäßrigen Aminoxidlösung eingestellt wird, indem das gebrauchte Fällbad mit einem mit alkalischen Gruppen modifizierten Adsorberharz, anschließend mit einem alkalischen Anionenaustauscher, welcher eine alkalische Substanz in das Fällbad einbringt, und zumindest teilweise mit einem sauren Kationenaustauscher in Kontakt gebracht wird.

8. Verfahren nach Anspruch 7, **dadurch gekennzeichnet, daß** ein Anionenaustauscher eingesetzt wird, der ausschließlich mit Alkalilauge und/oder flüchtigen organischen Säuren regeneriert wurde.

9. Verfahren nach einem der Ansprüchen 6 oder 7, **dadurch gekennzeichnet, daß** vor, während oder nach dem Regenerieren des Fällbades eine antioxidativ wirkende Substanz eingebracht wird.

**Claims**

1. A process for producing cellulosic moulded bodies, which process comprises the following steps:

> (A) dissolving cellulose in an aqueous solution of a tertiary amine oxide, in particular N-methylmorpholine-N-oxide (NMMO), to form a mouldable cellulose solution,
> (B) moulding the cellulose solution and passing the moulded cellulose solution into an aqueous precipitation bath in which the cellulose is precipitated, as a result of which a moulded body and a spent precipitation bath are formed,
> (C) regenerating the spent precipitation bath, in which process a regenerated aqueous amine-oxide solution is formed which is reused in step (A) to dissolve cellulose,

**characterized in that**
a regenerated aqueous amine-oxide solution is used in step (A) which has a pH in a range whose upper limit and whose lower limit are defined, as a function of the concentration of tertiary amine oxide, by the equation

$$pH = -0.0015 \times A^2 + 0.2816 \times A + f,$$

where A is the concentration of tertiary amine oxide in the aqueous solution expressed as a percentage by mass of the aqueous solution and the condition

$$40\% \leq A \leq 86\%,$$

preferably

$$70\% \leq A \leq 80\%,$$

is fulfilled and f has the value 1.00 for the upper limit and the value -1.80, preferably -1.00, for the lower limit.

2. A process for producing cellulosic moulded bodies, which process comprises the following steps:

> (A) dissolving cellulose in an aqueous solution of a tertiary amine oxide, in particular N-methylmorpholine-N-oxide (NMMO), to form a mouldable cellulose solution,
> (B) moulding the cellulose solution and passing the moulded cellulose solution into an aqueous precipitation bath in which the cellulose is precipitated, as a result of which a moulded body and a spent precipitation bath are formed,
> (C) regenerating the spent precipitation bath, in which process a regenerated aqueous amine-oxide solution is formed which is reused in step (A) to dissolve cellulose,

**characterized in that**
a regenerated aqueous amine-oxide solution is used in step (A) which has a pH in a range between 11.5 and 13.5.

3. A process according to claim 1, **characterized in that** the regenerated aqueous amine-oxide solution which is used in step (A) has a pH in the region of 10.5 and 13.5.

4. A process according to claim 1, **characterized in that** the regenerated aqueous amine-oxide solution which is used in step (A) has a pH in the region of 11.5 and 13.5.

5. A process according to any of claims 1 to 2, **characterized in that** the regenerated aqueous amine-oxide solution which is used in step (A) has a pH in the region of 11.5 and 12.5.

6. A process according to any of claims 1 to 5, **characterized in that** the pH value of the regenerated aqueous amine-oxide solution is adjusted by bringing the spent precipitation bath into contact with an alkaline anion exchanger which introduces an alkaline substance into the precipitation bath and, if necessary, subsequently bringing it into contact with an acidic cationic exchanger.

7. A process according to any of claims 1 to 5, **characterized in that** the pH of the regenerated aqueous amine-oxide solution is adjusted by bringing the spent precipitation bath into contact with an adsorber resin modified with alkaline groups, then into contact with an alkaline anion exchanger which introduces an alkaline substance into the precipitation bath, and at least partially, into contact with an acidic cationic exchanger.

8. A process according to claim 7, **characterized in that** an anion exchanger is used which has been regenerated solely with alkali hydroxide solution and/or volatile organic acids.

9. A process according to any of claims 6 or 7, **characterized in that** a substance having antioxidant activity is introduced before, during or after the regeneration of the precipitation bath.

**Revendications**

1. Procédé de fabrication de corps façonnés cellulosiques, lequel procédé comprend les étapes suivantes :

(A) dissolution de cellulose dans une solution aqueuse d'un aminoxyde tertiaire, en particulier le N-oxyde de N-méthylmorpholine (NMMO), pour former une solution de cellulose façonnable,
(B) façonnage de la solution de cellulose et introduction de la solution de cellulose façonnée dans un bain de précipitation aqueux dans lequel la cellulose est précipitée, de sorte qu'il se forme un corps façonné et un bain de précipitation usé,
(C) régénération du bain de précipitation usé avec formation d'une solution aqueuse d'aminoxyde régénérée qui est utilisée de nouveau dans l'étape (A) pour la dissolution de la cellulose,

**caractérisé**
**en ce que** l'on utilise dans l'étape (A) une solution aqueuse d'aminoxyde régénérée qui présente un pH situé dans un domaine dont la limite supérieure et dont la limite inférieure sont définies, en fonction de la concentration de l'aminoxyde tertiaire, par l'équation

$$pH = -0{,}0015 \times A^2 + 0{,}2816 \times A + f$$

dans laquelle A est la concentration de l'aminoxyde tertiaire dans la solution aqueuse, exprimée en pourcentage en masse de la solution aqueuse, et remplit la condition

$$40\ \% \leq A \leq 86\ \%$$

de préférence

$$70\ \% \leq A \leq 80\ \%,$$

et f possède pour la limite supérieure la valeur 1,00 et pour la limite inférieure la valeur -1,80, de préférence -1,00.

2. Procédé de fabrication de corps façonnés cellulosiques, lequel procédé comprend les étapes suivantes :

(A) dissolution de cellulose dans une solution aqueuse d'un aminoxyde tertiaire, en particulier le N-oxyde de N-méthylmorpholine (NMMO), pour former une solution de cellulose façonnable,
(B) façonnage de la solution de cellulose et introduction de la solution de cellulose façonnée dans un bain de précipitation aqueux dans lequel la cellulose est précipitée, de sorte qu'il se forme un corps façonné et un bain de précipitation usé,
(C) régénération du bain de précipitation usé avec formation d'une solution aqueuse d'aminoxyde régénérée qui est utilisée de nouveau dans l'étape (A) pour la dissolution de la cellulose,

**caractérisé**
**en ce que** l'on utilise dans l'étape (A) une solution aqueuse d'aminoxyde régénérée qui présente un pH situé dans un domaine compris entre 11,5 et 13,5.

3. Procédé selon la revendication 1, **caractérisé en ce que** la solution aqueuse d'aminoxyde régénérée qui est utilisée dans l'étape (A) présente un pH situé dans le domaine de 10,5 à 13,5.

4. Procédé selon la revendication 1, **caractérisé en ce que** la solution aqueuse d'aminoxyde régénérée qui est utilisée dans l'étape (A) présente un pH situé dans le domaine de 11,5 à 13,5.

5. Procédé selon l'une des revendications 1 à 2, **caractérisé en ce que** la solution aqueuse d'aminoxyde régénérée qui est utilisée dans l'étape (A) présente un pH situé dans le domaine de 11,5 à 12,5.

6. Procédé selon l'une des revendications 1 à 5, **caractérisé en ce que** le pH de la solution aqueuse d'aminoxyde régénérée est ajusté par le fait que le bain de précipitation usé est mis en contact avec un échangeur d'anions alcalin qui introduit une substance alcaline dans le bain de précipitation et éventuellement ensuite avec un échangeur de cations acide.

7. Procédé selon l'une des revendications 1 à 5, **caractérisé en ce que** le pH de la solution aqueuse d'aminoxyde régénérée est ajusté par le fait que le bain de précipitation usé est mis en contact avec une résine adsorbante modifiée avec des groupes alcalins, puis avec un échangeur d'anions alcalin qui introduit une substance alcaline dans le bain de précipitation et au moins en partie avec un échangeur de cations acide.

8. Procédé selon la revendication 7, **caractérisé en ce que** l'on utilise un échangeur d'anions qui a été régénéré exclusivement avec de la lessive alcaline et/ou des acides organiques volatils.

9. Procédé selon l'une des revendications 6 et 7, **caractérisé en ce qu'**une substance à action antioxydante est introduite avant, pendant ou après la régénération du bain de précipitation.

Fig. 1

Fig. 2